# EUROPEAN PATENT APPLICATION

(11) **EP 4 623 804 A1**
(43) Date of publication of application: **01.10.2025**
(21) Application number: 25166091.6
(22) Date of filing: 25.03.2025
(51) Int. Cl.: A61B 3/15, A61B 3/00, A61B 3/11, A61B 3/113

(54) **OPHTHALMOLOGIC APPARATUS**

(30) Priority: 27.03.2024 JP 2024051186
(71) Applicant: TOPCON CORPORATION, Tokyo 174-8580 (JP)
(72) Inventor: OMIYA, Takeshi, Tokyo, 174-8580 (JP)
(74) Representative: Louis Pöhlau Lohrentz

(57) **Abstract**

An ophthalmologic apparatus (A) includes an optical system (50) that is configured to acquire ocular information of a subject eye (E) of an examinee; a face support (30, 33) that is configured to support a face of the examinee; a controller (60) that is configured to control at least the optical system (50) and the face support (30, 33). The ophthalmologic apparatus (A) further includes an event camera (80) that is installed at a position to capture an anterior ocular segment of the subject eye (E), the event camera (80) being configured to output only event data, in which a luminance change has exceeded a set threshold from pixel data of an image frame of the anterior ocular segment of the subject eye (E), and the event data being output together with information on coordinates and time as output information. The controller (60) includes a face movement monitoring processing portion (64) that is configured to extract inner canthus point (IC) and outer canthus point (OC) of the subject eye (E) by using the output information from the event camera (80) and to monitor a movement of the face of the examinee based on the extracted inner and outer canthus points (IC, OC).

## Description

### FIELD OF THE INVENTION

The present invention relates to an ophthalmologic apparatus.

### BACKGROUND

JP2021-154105A proposes an ophthalmologic apparatus including a determination portion. The determination portion is configured to determine whether a face support including a chin rest properly supports the face of an examinee based on a detection result of a pupil image in each anterior ocular segment image determined by using a pupil image detection portion, to properly support the face of the examinee by the face support. JP7008131B2 proposes a method and apparatus for eye tracking using event camera data, which is applied to a head-mounted device and includes the determination of an eye tracking characteristic of a user based on light intensity data.

An ophthalmologic apparatus adjusts the positional relationship between the optical system and the subject eye through alignment, carries out subjective and objective examinations of the ocular characteristics of the subject eye, and captures subject eye images, such as ocular fundus images, when the examinee rests his/her chin on the chin rest. The examinee's face, with his/her chin resting on the chin rest, should remain still during alignment and ocular information acquisition. Accordingly, the ophthalmologic apparatus needs to check in real time whether the examinee's face, which rests on the chin rest, remains stationary or moves during alignment and ocular information acquisition. In this specification, the term "during ocular information acquisition" may include during the acquisition of the ocular characteristic values through subjective examination of the ocular characteristics of the subject eye, during acquisition of the ocular characteristic values through the objective examination of the ocular characteristics of the subject eye, and during the acquisition of the subject eye images by capturing images such as ocular fundus images.

In contrast, the technology disclosed in JP2021-154105A determines that the face is properly supported when the duration in which the conditions are met reaches or exceeds a certain period. The conditions include that the amount of face movement remains within a threshold value and that the rotation angle stays within a range corresponding to that of the fixation eye movement. Accordingly, the technology disclosed in JP2021-154105A requires detecting the position and shape of the pupil image, detecting the position and rotation angle of the subject eye, determining the detected face movement amount condition, determining the detected rotation angle condition, and determining the duration time condition. Thus, the technology disclosed in JP2021-154105A requires a process flow that includes checking the conditions of the examinee's face and the subject eye's pupil in advance, performing the alignment, and acquiring ocular information. As a result, it is impossible to check the movement of the examinee's face during the alignment and ocular information acquisition.

The technology disclosed in JP7008131B2 is an eye-tracking technology that detects the movement of the human pupil and tracks the visual line using an event camera. Accordingly, even if this technology is applied to an ophthalmologic apparatus, it cannot check the movement of the examinee's face during the alignment and ocular information acquisition. As a result, if the examinee's face moves during the alignment, the adjustment of the positional relationship between the optical system and the subject eye may take a long time or may fail. If the examinee's face moves during the measurement of eye characteristics as the ocular information of the subject eye, it may lead to low precision in acquired eye characteristics or failure in acquiring them. When capturing the subject eye images, such as the ocular fundus image as the ocular information of the subject eye, the movement of the examinee's face during the capturing may cause blur or flare in the image or failure in the capturing itself.

The present invention has been made in view of the above circumstances. An object of the present invention is to provide an ophthalmologic apparatus capable of quickly checking whether the examinee's face has moved during alignment and ocular information acquisition in real time.

### SUMMARY

According to one embodiment of the present invention, an ophthalmologic apparatus includes an optical system that is configured to acquire ocular information of a subject eye of an examinee; a face support that is configured to support a face of the examinee; and a controller that is configured to control at least the optical system and the face support. The ophthalmologic apparatus further includes an event camera that is installed at a position to capture an anterior ocular segment of the subject eye, the event camera being configured to output only event data, in which a luminance change has exceeded a set threshold from pixel data of an image frame of the anterior ocular segment of the subject eye, and the event data being output together with information on coordinates and time as output information. The controller includes a face movement monitoring processing portion that is configured to extract inner canthus point and outer canthus point of the subject eye by using the output information from the event camera and to monitor a movement of the face of the examinee based on the extracted inner and outer canthus points.

The ophthalmologic apparatus according to the present invention can quickly determine whether the examinee's face has moved during the alignment and the ocular information acquisition through real-time processing by monitoring the movement of the examinee's face based on the inner and outer canthus points extracted using the output information from the event camera.

### BRIEF DESCRIPTION OF DRAWINGS

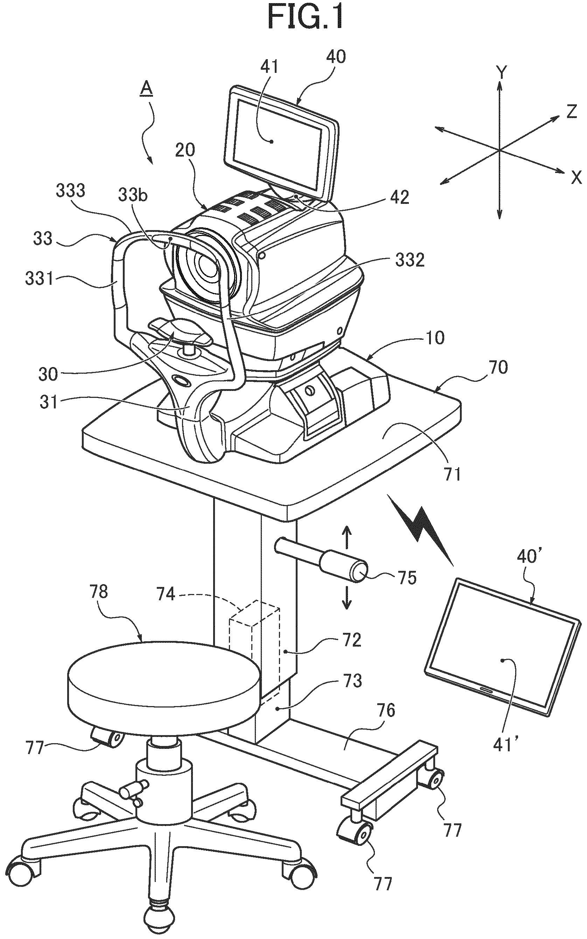

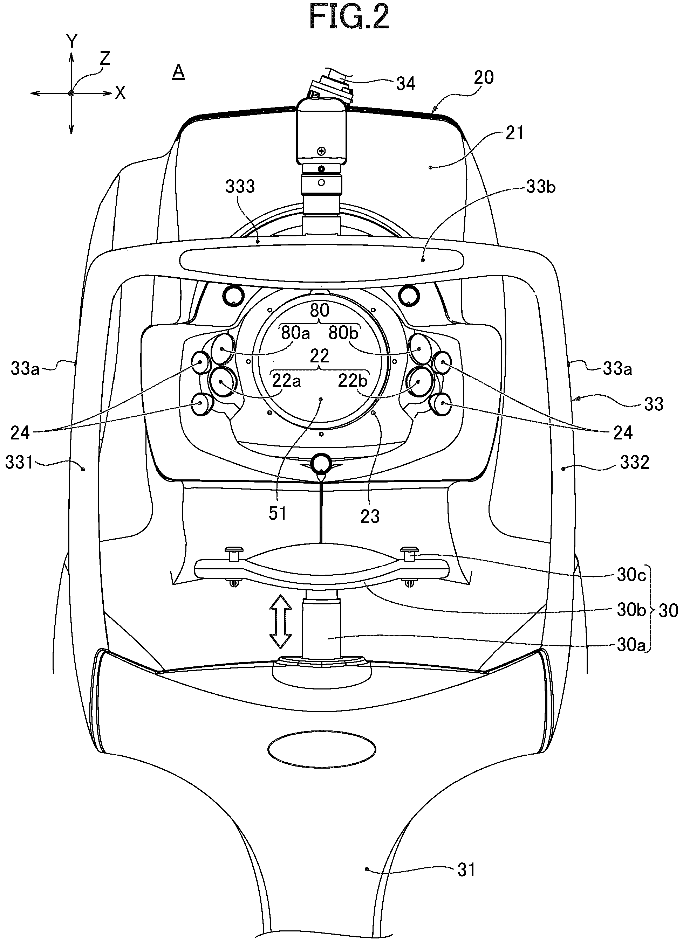

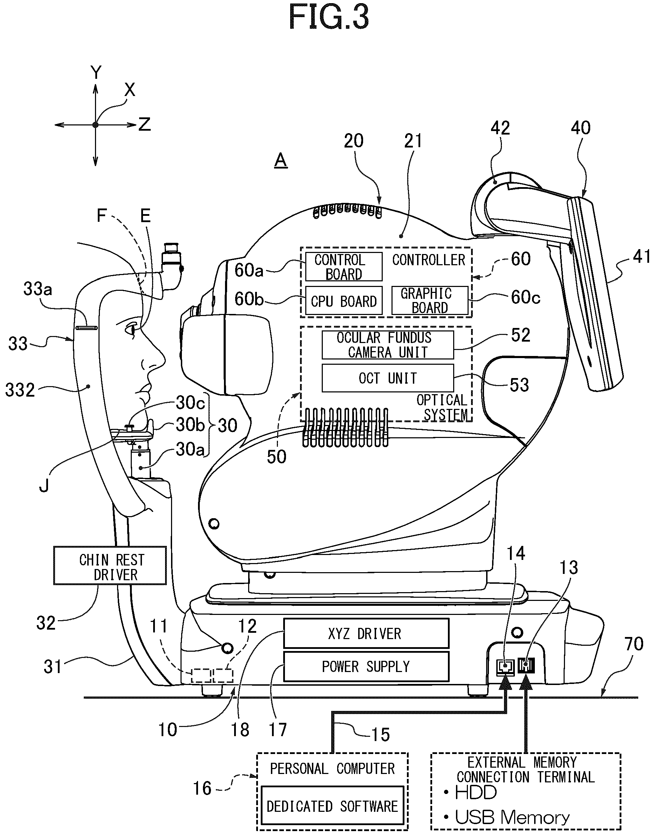

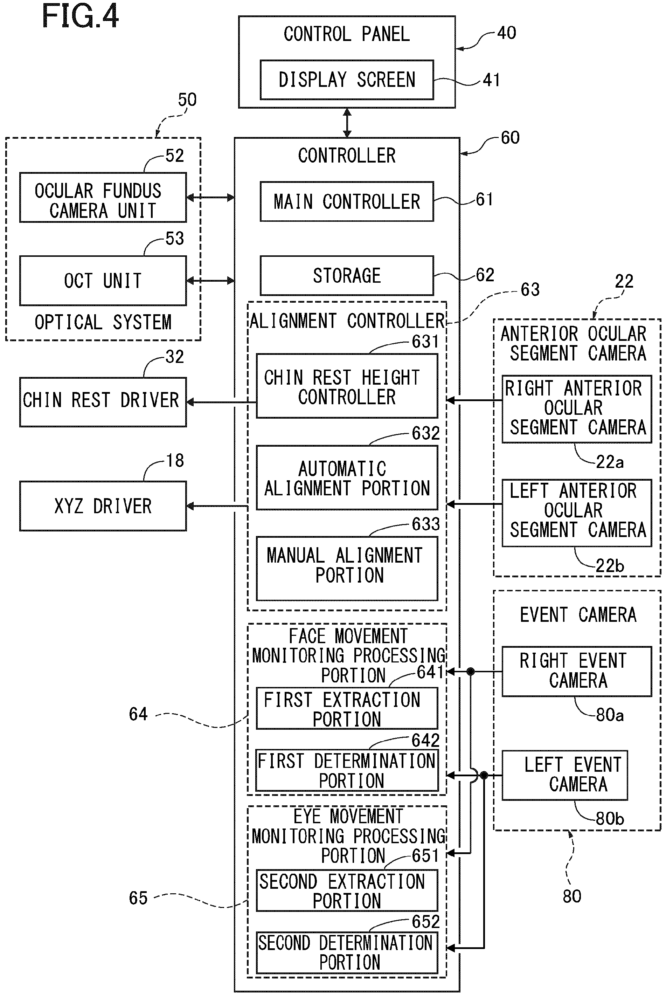

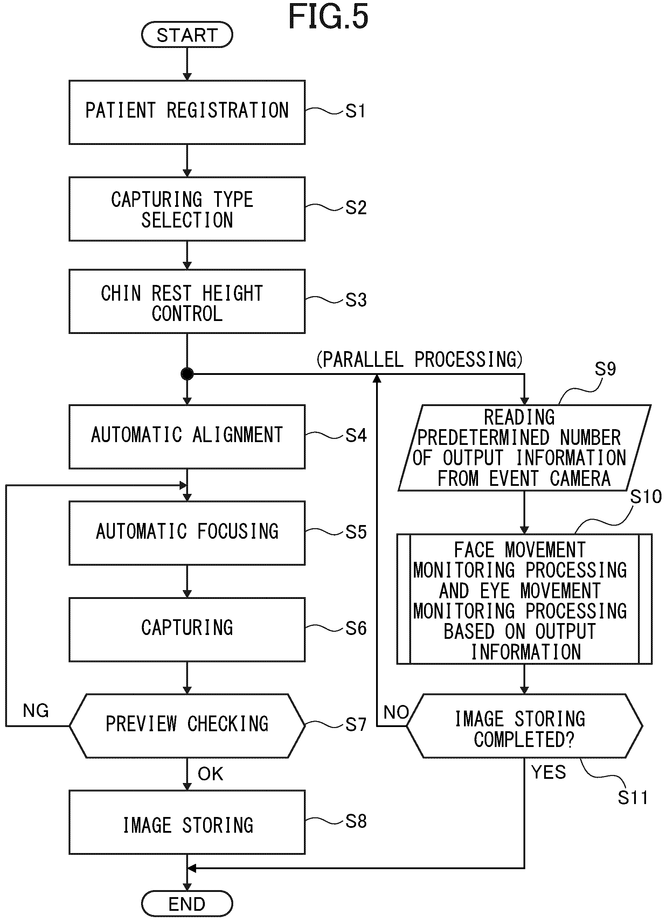

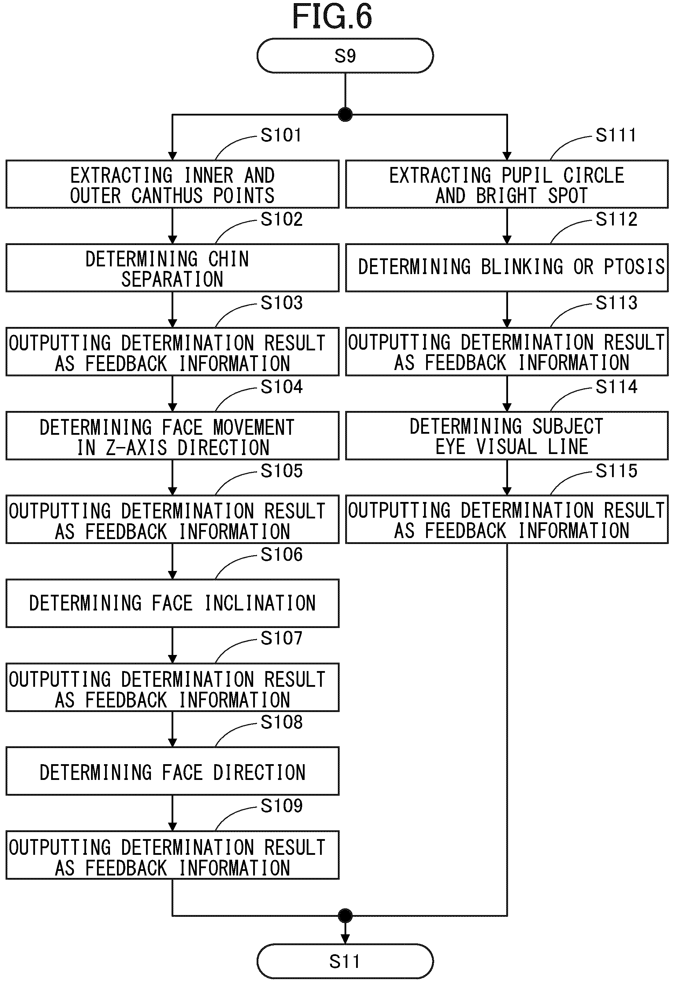

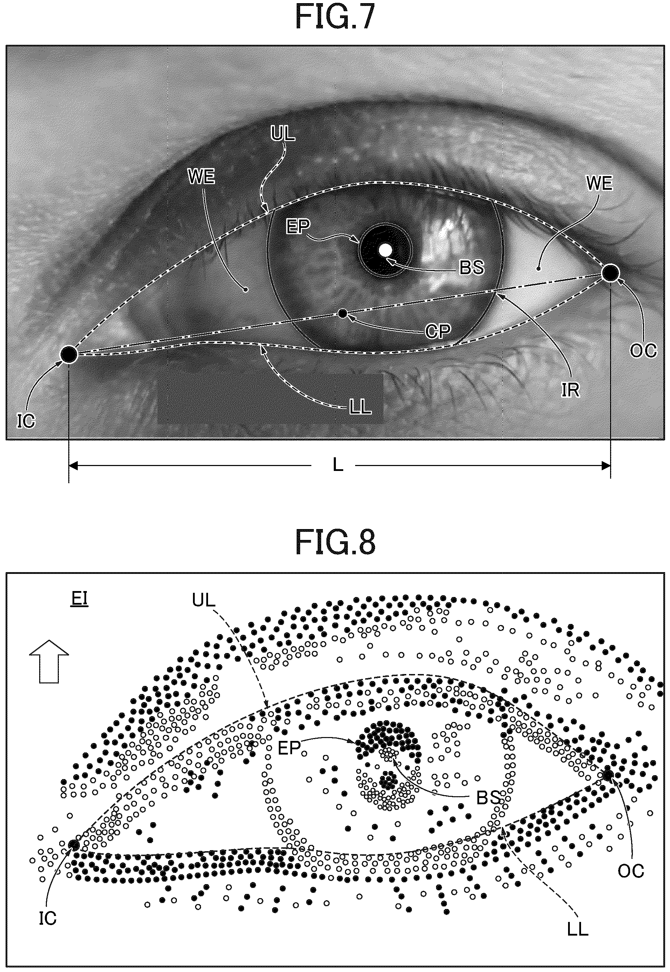

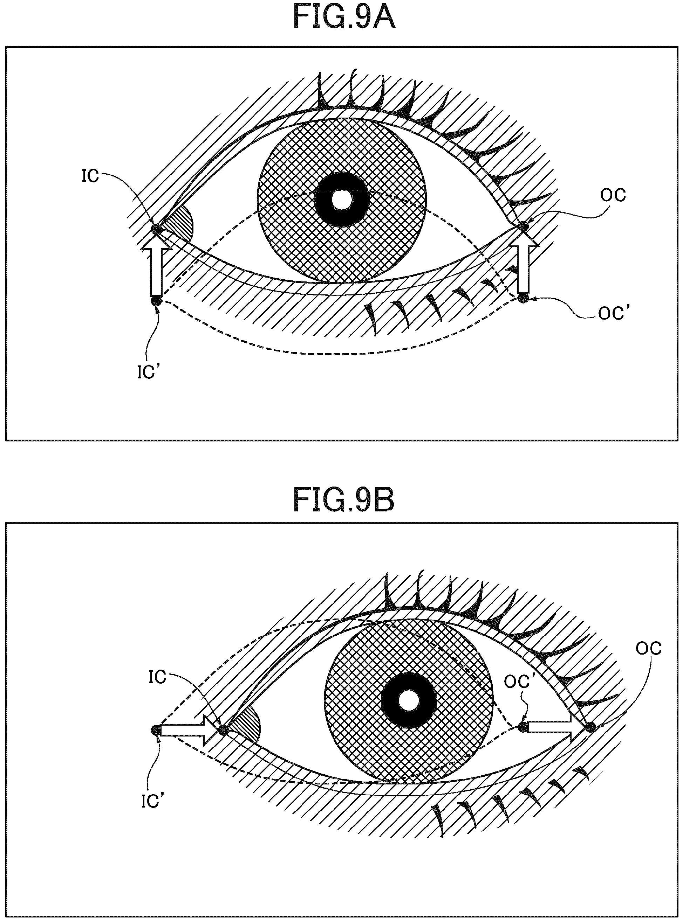

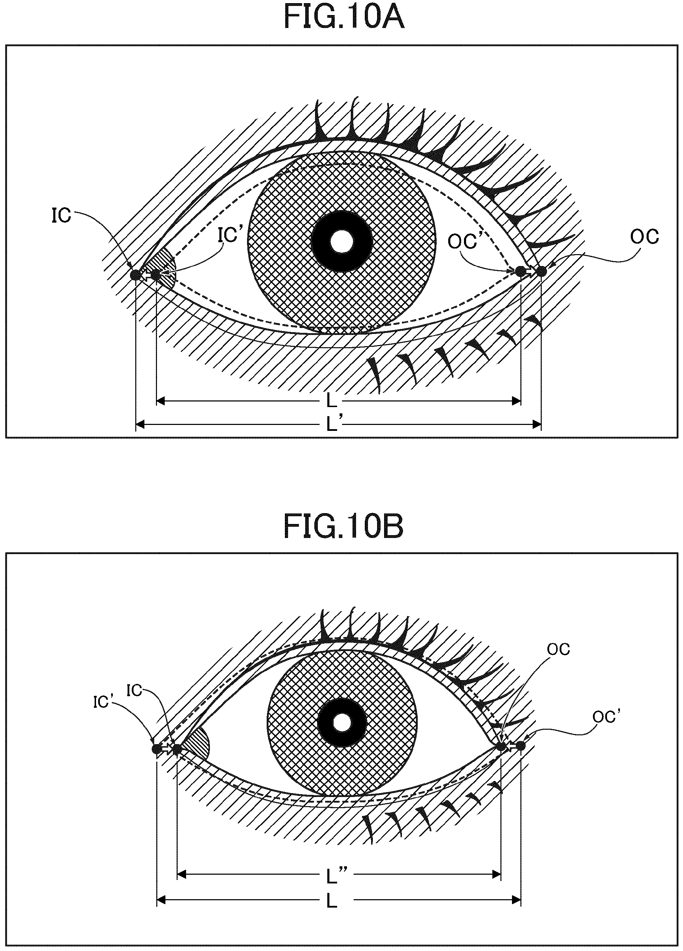

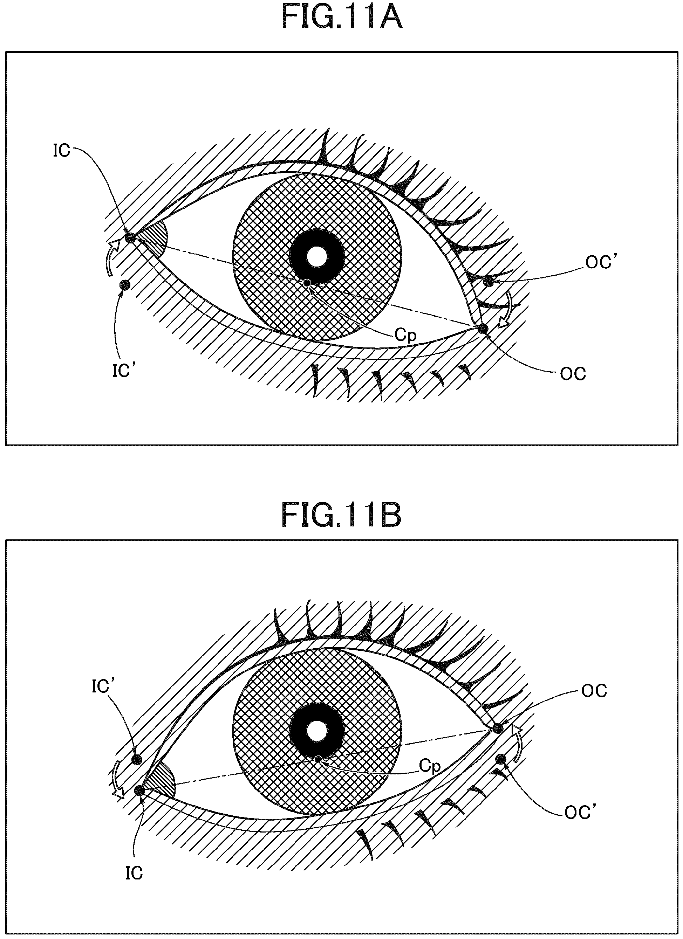

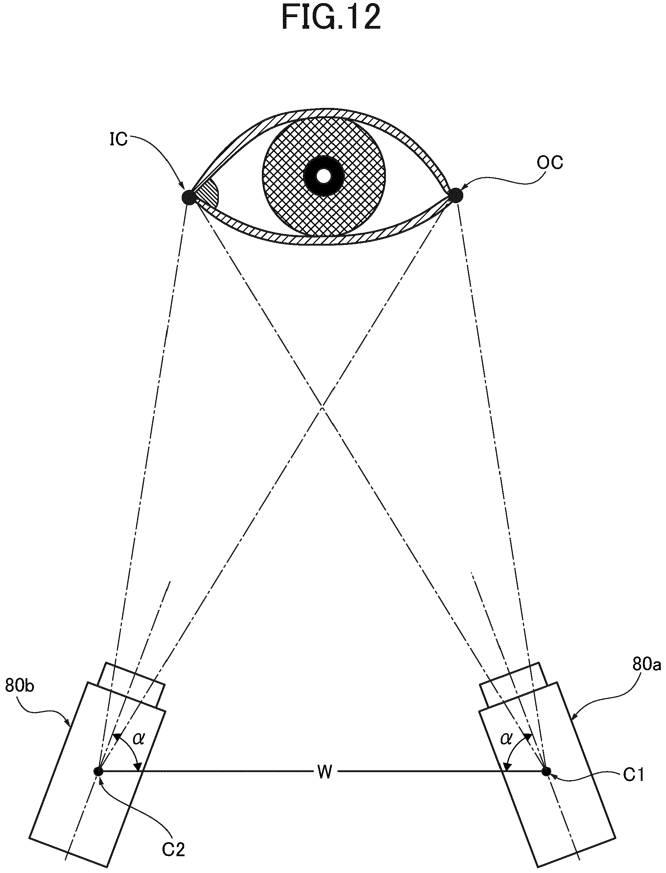

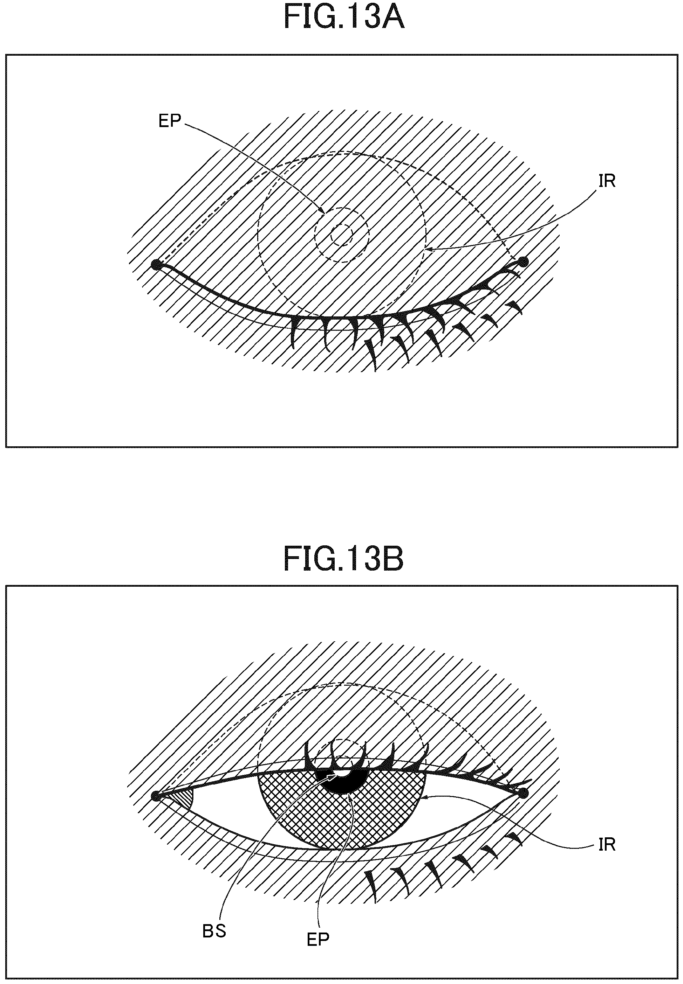

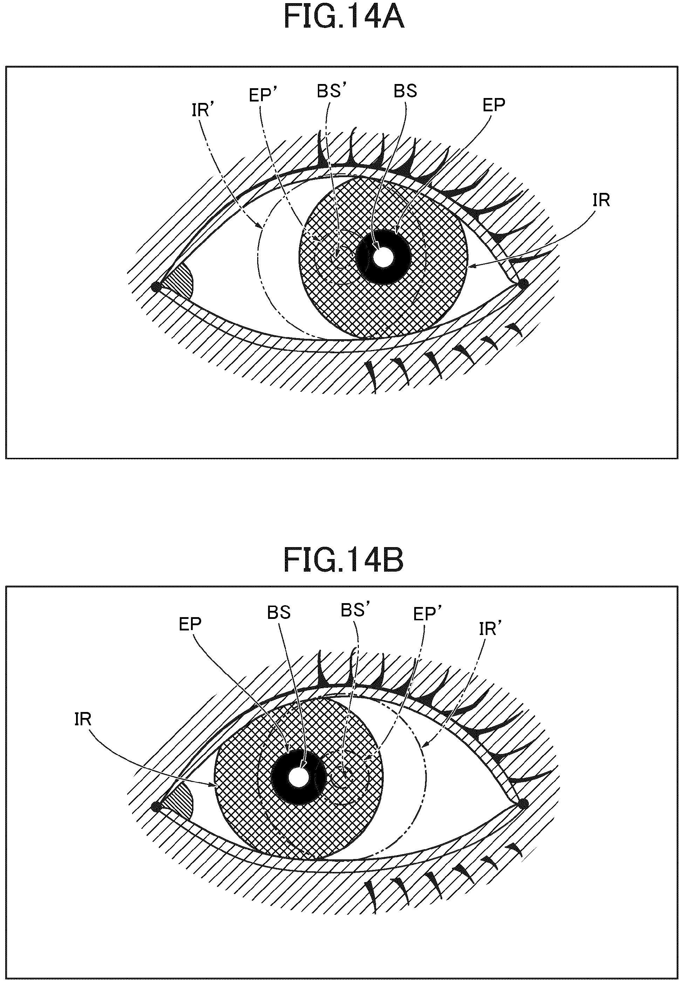

FIG. 1 is a perspective view showing the entire structure of an ophthalmologic system including an ophthalmologic apparatus according to a first embodiment. FIG. 2 is a front view showing the structure of the ophthalmologic apparatus according to the first embodiment, viewed from a chin rest side. FIG. 3 is a side view showing the ophthalmologic apparatus according to the first embodiment. FIG. 4 is a block diagram showing a control system configuration in the ophthalmologic apparatus according to the first embodiment. FIG. 5 is a flowchart showing the subject eye image capturing process in parallel with face and eye movement monitoring. FIG. 6 is a flowchart showing the face movement monitoring process and eye movement monitoring process. FIG. 7 is an example of anterior ocular segment images acquired by an anterior ocular segment camera. FIG. 8 is an example of event data acquired by an event camera. FIG. 9A is an explanatory view showing a first determination example when the face of the examinee has moved upward. FIG. 9B is a first determination example when the face of the examinee has moved sideways. FIG. 10A is a first determination example when the face of the examinee has moved forward. FIG. 10B is a first determination example when the face of the examinee has moved backward. FIG. 11A is a first determination example when the face of the examinee has tilted left. FIG. 11B is a first determination example when the face of the examinee has tilted right. FIG. 12 is an explanatory view showing face direction detection using the event cameras in a stereo arrangement. FIG. 13Ais an explanatory view showing a second determination example when the examinee has closed his/her upper eyelid by blinking. FIG. 13B is an explanatory view showing a second determination example when the subject eye has ptosis. FIG. 14A is an explanatory view showing a second determination example when the examinee oriented his/her visual line from forward to the left. FIG. 14B is an explanatory view showing a second determination example when the examinee oriented his/her visual line from forward to the right.

### DETAILED DESCRIPTION

With respect to the use of plural and/or singular terms herein, those having skill in the art can translate from the plural to the singular and/or from the singular to the plural as is appropriate to the context and/or application.
The various singular/plural permutations may be expressly set forth herein for the sake of clarity.

A configuration for implementing an ophthalmologic apparatus according to the present invention will be described based on the first embodiment shown in the drawings.

The ophthalmologic apparatus, which is applied to the first embodiment, is an apparatus that observes, captures, and records anterior ocular segment images, ocular fundus images, and ocular fundus tomographic images of the subject eyes and provides them as electronic images for diagnosis. When the ophthalmologic apparatus is positioned to face the subject eye, the X, Y, and Z in the drawings respectively indicate the X-axis as the left-right axis in the left-right direction (horizontal direction), the Y-axis as the vertical axis in the up-down direction (vertical direction), and the Z-axis as the front-back axis (depth direction) orthogonal to the X and Y axes.

### First Embodiment

The overall configuration of the apparatus will be described with reference to FIGS. 1 to 3. As shown in FIG. 1, an ophthalmologic apparatus A includes a pedestal 10, a body 20, a chin rest 30, a control panel 40, an optical system 50, a controller 60, an optical table 70, and an event camera 80.

The ophthalmologic apparatus A is called a three-dimensional ocular fundus imaging apparatus and includes an ocular fundus camera that acquires ocular fundus images of a subject eye E and an optical coherence tomograph (OCT) that acquires ocular fundus tomographic images of the subject eye E. Herein, the ocular fundus camera refers to a camera that captures images of the ocular fundus, including the retina, optic nerve, and capillaries at the back of the subject eye E, and acquires the resulting ocular fundus images. The OCT is an optical coherence tomograph that captures cross-sectional images of the retina at the ocular fundus of the subject eye E by utilizing light interference and acquires the resulting ocular fundus tomographic images.

The pedestal 10 is placed on the optical table 70, which is height-adjustable in the Y-axis direction. The pedestal 10 supports the body 20 at its top surface, allowing it to move in three axis directions of the X, Y, and Z axes. The pedestal 10 has a chin rest 30 at its front surface. The pedestal 10 is provided with a power switch 11, a power inlet 12, a universal serial bus (USB) terminal 13, and a local area network (LAN) terminal 14 at its both side surfaces. As shown in FIG. 3, the USB terminal 13 serves as an external memory connection port, allowing the connection of external storage devices such as a hard disk drive (HDD) and a USB memory device. The LAN terminal 14 is used to connect a personal computer 16, in which dedicated software is installed, via a LAN cable 15.

As shown in FIG. 3, the pedestal 10 includes a power supply 17 and an XYZ driver 18, both of which are built therein. The power supply 17 includes components such as the power switch 11, the power inlet 12, the USB terminal 13, and the LAN terminal 14. The XYZ driver 18 is a motor actuator that includes a motor and a motor drive circuit and drives the body 20 in the X-axis, Y-axis, and Z-axis directions (three-dimensional direction) when moving the body 20 relative to the pedestal 10 during alignment control.

The body 20 is installed to be movable in the X-axis, Y-axis, and Z-axis directions using the XYZ driver 18 relative to the pedestal 10, to which the chin rest 30 is fixed. The body 20 includes the optical system 50 within a body cover 21 that encloses the entire structure. The optical system 50 acquires ocular information of the subject eye E while the examinee's chin J is positioned on the chin rest 30. The control panel 40 is positioned at the top of the back surface of the body cover 21, as shown in FIGS. 1 and 3. As shown in FIG. 3, the body 20 includes the controller 60 within the inner space of the body cover 21, in addition to the optical system 50.

As shown in FIG. 2, the body cover 21 includes, at its front surface, an objective lens 51 of the optical system 50, which faces the subject eye E. The objective lens 51 is surrounded by an anterior ocular segment camera 22, an event camera 80, peripheral fixation lights 23, and anterior ocular segment observation filters 24 at its peripheral portion.

The anterior ocular segment camera 22 is configured to acquire anterior ocular segment images by capturing the anterior ocular segment of the examinee. The anterior ocular segment camera 22 includes two, right and left, anterior ocular segment cameras 22a, 22b. The right anterior ocular segment camera 22a and the left anterior ocular segment camera 22b are arranged in a stereo configuration on both sides of the objective lens 51, with their optical axes inclined toward the anterior ocular segment of the subject eye E, which is the target of capturing. The right and left anterior ocular segment cameras 22a, 22b respectively acquire a right-side anterior ocular segment image and a left-side anterior ocular segment image by extracting a portion of the examinee's face resting on the chin rest 30, in accordance with the selection of the subject eye E and the angle of view at that time. Furthermore, the right and left anterior ocular segment cameras 22a, 22b are positioned at a predetermined width from each other in the X-axis direction, and each have a specific estimated angle. Accordingly, the three-dimensional coordinate position of the subject eye E can be identified through a calculation process based on the two anterior ocular segment images.

Each of the peripheral fixation lights 23 is configured to fix the line of sight of the subject eye E by turning it on. The peripheral fixation lights 23 are equidistantly arranged at an outer peripheral position of the objective lens 51. In one embodiment, eight peripheral fixation lights 23 are equidistantly arranged, but the number of the peripheral fixation lights 23 is not limited to eight. Each of the anterior ocular segment observation filters 24 is configured to adjust light intensity during anterior ocular segment observation and anterior ocular segment optical coherence tomography (OCT). A plurality of filters is arranged in a vertical line at an outer position of the right anterior ocular segment camera 22a, and a plurality of filters are arranged in a vertical line at an outer position of the left anterior ocular segment camera 22b. In one embodiment, two filters are arranged in a vertical line at an outer position of the right anterior ocular segment camera 22a, and two filters are arranged in a vertical line at an outer position of the left anterior ocular segment camera 22b, making a total of four filters. The number of the anterior ocular segment observation filters 24 is not limited to four. The event camera 80 will be described in detail hereinafter.

The face support according to the first embodiment includes the chin rest 30 and a forehead rest 33. As shown in FIGS. 2 and 3, the chin rest 30 is configured to support the examinee's chin J and to be movable in the vertical direction relative to a chin rest holding portion 31 and the forehead rest 33, which are fixed to the pedestal 10. The chin rest 30 includes a lifting rod 30a, a chin rest base 30b, and chin rest paper stopping pins 30c. The lifting rod 30a is raised and lowered using a built-in chin rest driver 32. The chin rest base 30b is fixed at the top end of the lifting rod 30a. The chin rest paper stopping pins 30c are provided at both sides of the chin rest base 30b.

As shown in FIG. 2, the chin rest holding portion 31 is a T-shaped structure. The forehead rest 33 is fixed to both ends of the chin rest holding portion 31. The forehead rest 33 is configured to surround the examinee's face from three directions when the examinee's chin J rests on the chin rest 30. The forehead rest 33 includes a pair of vertical support portions 331, 332 and a horizontal support portion 333. The vertical support portions 331, 332 are fixed to the pedestal 10 and extend in the Y-axis direction. The horizontal support portion 333 extends between the upper ends of the vertical support portions 331, 332. Each of the vertical support portions 331, 332 includes an eye level line 33a at a position corresponding to the height center of a measurement section aperture for the objective lens 51. The eye level line 33a serves as a guide for adjusting the subject eyes E to the proper height position. The horizontal support portion 333 is provided with a removable forehead support portion 33b at the center of a first surface that contacts the examinee's forehead F. The forehead support portion 33b is made of silicon rubber or other similar materials. Furthermore, the horizontal support portion 333 is provided with an arm 34 at the center of a second surface opposite the first surface that contacts the examinee's forehead F. The arm 34 can be bent in multiple steps and has an external fixation target (not shown) at its distal end.

As shown in FIGS. 1 and 3, the control panel 40 is disposed at the top of the back surface of the body cover 21. The control panel 40 includes a display screen 41 that displays in color the anterior ocular segment images of the subject eye E captured using the anterior ocular segment camera 22, the anterior ocular segment observation images of the subject eye E from the optical system 50, and other related images. The display screen 41 includes a touch panel that allows an operator, such as an examiner, to input data by touching displayed button images and other interface elements with a finger. The control panel 40 is attached to the body 20 via a connecting support portion 42. The connecting support portion 42 includes a support structure that combines bending and rotary mechanisms. This support structure allows the display screen 41 to be positioned anywhere around the body 20 in the circumferential direction and enables free adjustment of its inclination angle.

The control panel 40 is used when the examiner is located next to the examinee during the examination of the eye characteristics. The connecting support portion 42 allows the display screen 41 to be positioned for easy operation by the examiner, regardless of the examiner's position around the ophthalmologic apparatus A. The examiner may use a remote operation tablet 40' shown in FIG. 1 for a remote examination of the eye characteristics. The remote operation tablet 40' includes a touch-panel display screen 41' with input functions equivalent to those of the control panel 40 and also has communication functions to connect with the body 20.

The optical system 50 acquires ocular information of the subject eye E when the examinee rests their chin on the chin rest 30. As shown in FIG. 3, the optical system 50 includes an ocular fundus camera unit 52 with the objective lens 51 and an optical coherence tomograph (OCT) unit 53. The ocular fundus camera unit 52 includes an illumination optical system and an imaging optical system. The ocular fundus camera unit 52 constitutes an ocular fundus camera, which acquires ocular fundus images using a lens and an image sensor, among other components. The OCT unit 53 constitutes the OCT, which acquires ocular fundus tomographic images of the subject eye E using a variable wavelength light source, a fiber coupler, and other components. The optical system 50 not only acquires ocular fundus images and ocular fundus tomographic images of the subject eye E, but also acquires anterior ocular segment observation images of the subject eye E.

The controller 60 controls various components of the apparatus, including the ocular fundus camera unit 52, the OCT unit 53, the chin rest 30, and the body 20, based on various input operations such as touch inputs on the display screen 41 of the control panel 40. As shown in FIG. 3, the controller 60 includes, as a hardware configuration, a control board 60a, a CPU board 60b, and a graphics board 60c. The controller 60 will be described in detail later.

As shown in FIG. 1, the optical table 70 includes a top plate 71 for placing the ophthalmologic apparatus A, a top plate support 72, a caster support 73, a table lifting mechanism 74, a lifting lever 75, a caster base 76, and casters 77. The table lifting mechanism 74 is disposed inside the top plate support 72 and the caster support 73 and is configured to move the top plate support 72 up and down relative to the caster support 73. The lifting lever 75 is a manually operated component configured to raise and lower the top plate 71, which holds the ophthalmologic apparatus A, and the top plate support 72 by manual operation. The stroke of the top plate 71 is set to a predetermined amount. For example, the predetermined amount may be 200mm or approximately 200 mm but is not limited to these values. The ophthalmologic apparatus A measures the examinee's eye characteristics while the examinee is seated on a stool 78 with an adjustable seat height. Therefore, the optical table 70 is set at a height suitable for seated imaging. The ophthalmologic apparatus A can also be used for imaging while the examinee is standing. In this case, a table that does not require the stool 78 and has a height suitable for standing measurements is used as the optical table 70.

The event camera 80 is installed at a position to capture the anterior ocular segment of the subject eye E. The event camera 80 is configured to output event data, in which luminance changes exceed a set threshold from pixel data of an image frame of the anterior ocular segment of the subject eye E. The event camera 80 outputs event data along with coordinate and time information. The event data includes plus events (bright areas) and minus events (dark areas). The plus events (bright areas) are output when the luminance increase exceeds a set positive threshold, while the minus events (dark areas) are output when the luminance decrease exceeds a set negative threshold. The event camera 80 includes two event cameras, a right event camera 80a and a left event camera 80b. The event cameras 80a, 80b are arranged in a stereo configuration on both sides of the objective lens 51, with their lens optical axes inclined toward the anterior ocular segment of the subject eye E, which is the target of capturing. The right and left event cameras 80a, 80b are positioned above the right-side and left-side anterior ocular segment cameras 22a, 22b, respectively. The right and left event cameras 80a, 80b are arranged with a specific width W between them in the X-axis direction, and each has a specific estimated angle α (see FIG. 12).

The configuration of the control system will be described with reference to FIG. 4. As shown in FIG. 4, a control system of the ophthalmologic apparatus A includes the control panel 40 (display screen 41), the optical system 50 (ocular fundus camera unit 52 and OCT unit 53), and the controller 60.

The controller 60 includes a main controller 61, a storage 62, an alignment controller 63, a face movement monitoring processing portion 64, and an eye movement monitoring processing portion 65. The main controller 61 is configured to control the ocular fundus camera unit 52 and the OCT unit 53. The storage 62 is configured to store required data.

The alignment controller 63 controls the alignment to adjust the positional relationship between the optical system 50 and the subject eye E while the examinee sits in front of the apparatus and rests their chin on the chin rest 30. The alignment controller 63 receives anterior ocular segment images from the anterior ocular segment cameras (i.e., the right-side anterior ocular segment camera 22a and the left-side anterior ocular segment camera 22b), which are arranged in a stereo configuration, and outputs drive commands to the XYZ driver 18 or the chin rest driver 32. The chin rest driver 32 moves the examinee's face in the Y-axis direction for the chin rest height control. The XYZ driver 18 adjusts the position in three-axis directions through automatic or manual alignment. As shown in FIG. 4, the alignment controller 63 includes a chin rest height controller 631, an automatic alignment portion 632, and a manual alignment portion 633.

The chin rest height controller 631 controls the output of drive commands to the chin rest driver 32 to align the position of the subject eye E with the height position of the eye level lines 33a while the examinee's chin J rests on the chin rest 30 and their forehead F contacts the forehead support portion 33b.

The automatic alignment portion 632 performs the automatic alignment relative to the pupil based on the anterior ocular segment image acquired by the anterior ocular segment camera 22. The automatic alignment relative to the pupil is achieved by automatically adjusting the captured eyes so that the pupil marks in the two anterior ocular segment images displayed on the video area of the display screen 41 coincide. The automatic adjustment of the captured eyes controls the XYZ alignment adjustment to overlap the two pupil marks at the center of the anterior ocular segment image by driving the XYZ driver 18.

The manual alignment portion 633 activates the manual alignment relative to the pupil when the elapsed time from the start of the automatic alignment reaches a preset time limit before completing the automatic alignment or when the examiner voluntarily selects manual operation. In the manual alignment relative to the pupil, tapping the manual mode button displayed on the automatic alignment screen stops the automatic adjustment of the captured eyes and switches to manual adjustment mode. In the manual adjustment mode, the examiner manually taps the two pupil marks in the anterior ocular segment images displayed on the display screen 41. The examiner's tap operation adjusts the XYZ alignment by driving the XYZ driver 18 so that the two pupil marks overlap at the center of the anterior ocular segment image.

The face movement monitoring processing portion 64 extracts the inner and outer canthus points of the subject eye E using the output information from the event camera 80 and monitors the movement of the examinee's face based on these points. The face movement monitoring processing portion 64 includes a first extraction portion 641 and a first determination portion 642. The first extraction portion 641 extracts the inner and outer canthus points of the subject eye using the output information from the event camera 80. The first determination portion 642 determines the movement of the examinee's face based on the extracted inner and outer canthus points. In addition to determining the movement of the examinee's face, the first determination portion 642 determines the direction of the examinee's face based on the extraction results of the inner and outer canthus points captured by the right and left event cameras 80a, 80b, which are arranged in the stereo configuration.

The eye movement monitoring processing portion 65 extracts a pupil circle, which represents the shape of the pupil of the subject eye E, and a bright spot projected onto the cornea of the subject eye E by the optical system 50, using the output information from the event camera 80, and monitors the movement of the subject eye E based on the extracted pupil circle and the bright spot. The eye movement monitoring processing portion 65 includes a second extraction portion 651 and a second determination portion 652. The second extraction portion 651 extracts the pupil circle and the bright spot of the subject eye E using the output information from the event camera 80. The second determination portion 652 determines the movement of the subject eye E based on the extracted pupil circle and bright spot.

Each of the face movement monitoring processing portion 64 and the eye movement monitoring processing portion 65 is preferably implemented using a field-programmable gate array (FPGA), which can be adapted to the event camera 80 in edge computing technology, where the ophthalmologic apparatus A processes data. The FPGA is a device that can be immediately reconfigured on-site using hardware description language (HDL), even if there is an error in the logic circuit design. Thus, when the event camera 80 is applied to the ophthalmologic apparatus A, the event camera 80 can be easily adapted to monitor and determine the movement of the examinee's face and the movement of the subject eye E.

The process for capturing the subject eye image, such as the anterior ocular segment image, the ocular fundus image, or the ocular fundus tomographic image, in parallel with monitoring face and eye movements by the controller 60 will be described with reference to FIG. 5. The capturing process starts when the ophthalmologic apparatus A determines the examinee after the power switch is turned on.

In Step S1, the examiner registers the patient (examinee) using a name or patient ID that identifies the patient. The patient ID is an identification number used to manage the examinee's personal information related to the ophthalmologic examination and may include age, gender, and previous examination information for follow-up.

In Step S2, the examiner selects the capturing type. The examiner selects one of the capturing modes using the touch operation on the capturing icon selection screen displayed on the display screen 41 of the control panel 40. For example, the capturing modes include an anterior ocular segment image capturing mode, an ocular fundus image capturing mode, an ocular fundus tomographic image capturing mode, and other modes. In Step S2, the examiner also selects the capturing eye using the touch operation to the display button.

In Step S3, the chin rest height controller 631 of the alignment controller 63 controls the height of the chin rest. The height of the top plate 71 of the optical table 70 or the stool 78 is adjusted to allow the examinee to rest their chin J on the chin rest 30 in a comfortable posture. Then, the examiner instructs the examinee to place their chin J on the chin rest 30, and the process starts after the examinee does so. In Step S3, the examiner adjusts the height of the chin rest 30 by pressing the chin rest vertical movement button displayed on the display screen 41 to align the position of the outer canthi of the subject eyes E with the height positions of the eye level lines 33a marked on the vertical support portions 331, 332 of the chin rest 30. After completing the chin rest height adjustment in Step S3, the examiner instructs the examinee to press their forehead F against the forehead support portion 33b.

In Step S4, the automatic alignment portion 632 of the alignment controller 63 performs automatic alignment (automatic adjustment) relative to the pupil of the captured eye. If the automatic alignment takes too long or if the examiner chooses to switch to manual alignment (manual adjustment) relative to the pupil, the examiner may do so using the manual alignment portion 633 instead of automatic alignment.

In Step S5, the main controller 61 performs automatic focusing, automatically adjusting the focus. In the anterior ocular segment image capturing mode, the focus is adjusted relative to the anterior ocular segment of the subject eye E. In the ocular fundus image capturing mode and the ocular fundus tomographic image capturing mode, the focus is adjusted relative to the ocular fundus of the subject eye E.

In Step S6, the examiner instructs to capture a subject eye image. For example, the subject eye image includes an anterior ocular segment image, an ocular fundus image, or an ocular fundus tomographic image. The examiner taps the OK button on the capturing screen displayed on the display screen 41 to confirm the current capture and proceed to the next capture after the completion of the current capture. A preview of the captured image is displayed after each capture.

In Step S7, the examiner checks the preview of the captured image and determines whether it is acceptable (OK) or not (NG). If the examiner determines the preview is acceptable (OK) in Step S7, the process proceeds to Step S8. If the examiner determines the preview is not acceptable (NG) in Step S7, the process returns to Step S5 to repeat the automatic focusing, followed by capturing in Step S6, until the preview is determined to be acceptable (OK).

In Step S8, the examiner instructs to store the captured image in the storage 62. When the examiner taps the store button on the capturing screen displayed on the display screen 41, the captured image at that moment is stored in the storage 62. After storing is completed, the process proceeds to END.

In Step S9, the face movement monitoring processing portion 64 and the eye movement monitoring processing portion 65 of the controller 60 read a predetermined amount of output information from the event camera 80. For example, the output information includes information such as event data, position coordinates, and time. The predetermined amount of output information refers to the quantity of output information acquired within a predetermined period for determining the movements of the examinee's face and eye based on the output information from the event camera 80. The sequential flow proceeding through Step S9, Step S10, and Step S11 is executed in parallel with the sequential flow of operations from Step S4, which includes the automatic alignment, the automatic focusing, and the capturing.

In Step S10, the face movement monitoring processing portion 64 and the eye movement monitoring processing portion 65 of the controller 60 respectively monitor the face movement and the eye movement based on the predetermined amount of the output information read from the event camera 80. The monitoring of the face movement and the eye movement will be described in detail later.

In Step S11, the face movement monitoring processing portion 64 and the eye movement monitoring processing portion 65 of the controller 60 determine whether storing the captured image in the storage 62 has been completed. If the storage has not been completed, the process returns to Step S9 to read a new predetermined amount of output information and then repeat the face movement monitoring and the eye movement monitoring. If the storage has been completed in Step S11, the process proceeds to END.

The face movement monitoring process and the eye movement monitoring process, which are executed in Step S10 of FIG. 5, will be described with reference to FIG. 6. In the subject eye E, the inner canthus point is denoted as IC, the outer canthus point as OC, the upper eyelid line as UL, the lower eyelid line as LL, the sclera (white area) as WE, the cornea (black area) as IR, the pupil circle as EP, the bright spot as BS, the distance between the inner and outer canthus points (IC and OC) as L, and the center portion of the subject eye E as CP (see FIG. 7).

In Step S101, the first extraction portion 641 of the face movement monitoring processing portion 64 extracts the inner canthus point IC and the outer canthus point OC of the subject eye E. In Step S101, the first extraction portion 641 extracts the upper eyelid line UL and the lower eyelid line LL using the output information from the event camera 80, when the face of the examinee has moved. At the upper and lower eyelid lines UL, LL, the luminance changes along the line segments of the upper and lower eyelids of the subject eye E, based on a luminance difference between each of the upper and lower eyelids and the sclera (white area) WE of the subject eye E. Then, the first extraction portion 641 extracts the inner and outer intersection points of the upper and lower eyelid lines UL, LL as the inner and outer canthus points IC, OC, respectively.

In Step S102, the first determination portion 642 of the face movement monitoring processing portion 64 determines whether the examinee's face has moved away from the face support (chin rest 30 and forehead rest 33). In Step S102, the first determination portion 642 monitors the movement of the extracted inner and outer canthus points IC, OC and determines that the examinee's chin J has moved away from the chin rest 30 and that the face has moved upward, when the inner and outer canthus points IC, OC move in the same upward direction on the two-dimensional coordinate plane defined by the X-axis and the Y-axis. In Step S102, the first determination portion 642 monitors the movement of the extracted inner and outer canthus points IC, OC and determines that the examinee's face has moved away from both the chin rest 30 and the forehead rest 33 and has moved in the lateral direction when the inner and outer canthus points IC, OC move in the same lateral direction on the two-dimensional coordinate plane defined by the X-axis and the Y-axis.

In Step S103, the first determination portion 642 outputs the determination result as feedback information indicating that the examinee's chin J has moved away from the chin rest. This feedback information may be displayed as a message on the display screen 41 of the control panel 40, indicating that "the examinee's chin has moved away from the chin rest." Alternatively, the feedback information may be output as an audio alert through a built-in speaker. Either or both of these notifications alert the examiner that the examinee's chin J is not properly positioned. Outputting the feedback information may include outputting a command to pause the alignment process and/or the capturing process in addition to the command to notify the examiner. When the first determination portion 642 determines that the examinee has properly positioned their chin J on the chin rest 30, a command is then issued to resume the process, and the pause is canceled.

In Step S104, the first determination portion 642 determines whether the examinee's face has moved in the Z-axis direction. In Step S104, the first determination portion 642 monitors changes in the distance L between the extracted inner and outer canthus points IC and OC. The first determination portion 642 determines that the examinee's face has moved closer to the apparatus along the Z-axis direction when an increase in distance L is monitored, and that the examinee's face has moved away from the apparatus along the Z-axis direction when a decrease in the distance L is monitored.

In Step S105, the first determination portion 642 outputs the determination result as feedback information when it determines that the examinee's face has moved in the Z-axis direction. This feedback information may be displayed as a message on the display screen 41 of the control panel 40, for example, to indicate that "the examinee's face is too close to the apparatus" or "the examinee's face is too far from the apparatus". Alternatively, the feedback information may be output as an audio alert through the built-in speaker. Either or both of these notifications may alert the examiner that the examinee's face has moved in the Z-axis direction. Similar to Step S103, outputting the feedback information may include outputting a command to pause the alignment process and/or the capturing process in addition to the command to notify the examiner.

In Step S106, the first determination portion 642 determines whether the examinee's face has tilted. In Step S106, the first determination portion 642 monitors the movement of the extracted inner and outer canthus points IC, OC relative to the center portion CP of the subject eye E. When the first determination portion 642 determines the rotational movement of the inner and outer canthus points IC, OC about the center portion CP, it determines that the examinee's face has tilted in that direction.

In Step S107, the first determination portion 642 outputs the determination result as feedback information when it determines that the examinee's face has tilted. This feedback information may be displayed as a message on the display screen 41 of the control panel 40, for example, to indicate that "the examinee's face has tilted to the right" or "the examinee's face has tilted to the left." Alternatively, the feedback information may be output as an audio alert through the built-in speaker. Either or both of these notifications may alert the examiner that the examinee's face has tilted. Similar to Step S103, outputting the feedback information may include outputting a command to pause the alignment process and/or the capturing process in addition to the command to notify the examiner.

In Step S108, the first determination portion 642 determines the direction of the examinee's face. In Step S108, the first determination portion 642 calculates the three-dimensional position coordinates of the inner and outer canthus points IC, OC based on the extraction results from the right and left event cameras 80a, 80b, which are arranged in the stereo configuration. Then, the first determination portion 642 determines that the direction orthogonal to the straight line connecting the inner and outer canthus points IC, OC in a three-dimensional coordinate space represents the direction of the face.

In Step S109, the first determination portion 642 outputs the determination result as feedback information when it determines the direction of the examinee's face. This feedback information may be displayed as a message on the display screen 41, for example, to indicate whether "the examinee's face is facing forward" or "the examinee's face is facing diagonally to the right." Alternatively, the feedback information may be output as an audio alert through a built-in speaker. Either or both of these notifications may make the examiner aware of the direction of the examinee's face. In some cases, capturing and/or measurement may be performed with the examinee's face in a diagonal position, deviated from the frontal orientation. Accordingly, a command to pause the alignment process and/or the capturing process is not automatically output. Instead, the examiner, who is made aware of the direction of the examinee's face, will decide whether or not to pause the process.

In Step S111, the second extraction portion 651 of the eye movement monitoring processing portion 65 extracts the pupil circle EP, which represents the shape of the pupil of the subject eye, and the bright spot BS projected onto the cornea IR of the subject eye E by the optical system 50, using the output information from the event camera 80. The bright spot BS is an alignment bright spot formed by the reflection of a parallel light flux projected onto the cornea IR of the subject eye E by the alignment system of the optical system 50. With regard to the pupil circle EP, the second extraction portion 651 extracts a contour area where the luminance change occurs due to a luminance difference between the pupil and the cornea (black area) IR of the subject eye E, using the output information from the event camera 80, when the position of the pupil with the minimum luminance changes. With regard to the bright spot BS, the second extraction portion 651 extracts a spot area where the luminance change occurs due to its positional shift in response to the luminance change caused by the movement of the pupil when the direction of the subject eye E's visual line changes. The amount of the positional change of the bright spot BS does not coincide with but is smaller than that of the pupil's positional change.

In Step S112, the second determination portion 652 determines whether the subject eye E has blinked or has ptosis. In Step S112, the second determination portion 652 monitors the shape of the extracted pupil circle EP and bright spot BS. Then, the second determination portion 652 determines that the subject eye E has blinked when both the pupil circle EP and the bright spot BS disappear for a moment. The second determination portion 652 determines that the subject eye E has ptosis when the pupil circle EP remains at least partially missing.

In Step S113, the second determination portion 652 outputs the determination result as feedback information when it determines that the subject eye E has blinked or has ptosis. This feedback information may be displayed as a message on the display screen 41 of the control panel 40, for example, to indicate that "the eye has blinked" or "the eyelid is partially open." Alternatively, it may be output as an audio alert through the built-in speaker. Either or both of these notifications may make the examiner aware that the subject eye E has blinked or that the eyelid is partially open. The examiner can respond to these cases by instructing the examinee with the message "Please refrain from blinking for a while" or helping the examinee. Accordingly, a command to pause the alignment process and/or the capturing process is not automatically output.

In Step S114, the second determination portion 652 determines the visual line of the subject eye E. In Step S114, the second determination portion 652 monitors the positional changes of the extracted pupil circle EP and bright spot BS and determines the visual line of the subject eye E based on these positional changes. In Step S114, the second determination portion 652 determines the visual line of the subject eye E by identifying the direction of the positional change of the pupil circle EP as the direction of the visual line shift.

In Step S115, the second determination portion 652 outputs the determination result as feedback information when it determines the visual line of the subject eye E. This feedback information may be displayed as a message on the display screen 41 of the control panel 40, for example, to indicate that "the visual line is facing forward" or "the visual line has shifted laterally." Alternatively, it may be output as an audio alert through the built-in speaker. Either or both of these notifications may make the examiner aware of the visual line of the subject eye E. The examiner can respond to these cases by instructing the examinee with a message such as "Please align your visual line to the front." Accordingly, a command to pause the alignment process and/or the capturing process is not automatically output.

The comparison between the anterior ocular segment image and the event camera image will be described with reference to FIGS. 7 and 8. An anterior ocular segment image FI shown in FIG. 7 is an image captured by the anterior ocular segment camera 22. If the upper eyelid line UL and the lower eyelid line LL have been drawn on the subject eye E in this anterior ocular segment image FI, the inner intersection point of the two intersection points is designated as the inner canthus point IC, and the outer intersection point is designated as the outer canthus point OC. The area enclosed by the upper and lower eyelid lines UL, LL includes the cornea IR as a black area, the sclera WE as a white area on both sides of the cornea IR, and the pupil circle EP as the central area of the cornea IR. The bright spot BS is a spot projected onto the cornea IR as an alignment bright spot. For example, when the subject eye E faces forward, it is located at the center position of the pupil circle EP. The distance L is defined as the distance between the inner and outer canthus points IC, OC. The center portion CP of the subject eye E is defined as the midpoint of the straight line segment connecting the inner and outer canthus points IC, OC.

The pupil circle EP is inherently dark. In contrast, the sclera WE is inherently bright. The characteristic that the pupil circle EP is dark and the sclera WE is bright is common to all human races. Accordingly, the pupil circle EP has a clear contour due to the luminance difference therebetween. Each of the upper and lower eyelid lines UL, LL appears as a distinct line segment due to the luminance difference. The bright spot BS appears as a distinct spot due to the luminance difference.

For example, when the face of the examinee moves upward, the anterior ocular segment image frame of the subject eye E from the event camera 80 is converted into an event data image EI, as shown in FIG. 8. In the event data image EI, when contours, line segments, points, and other features with luminance differences move, plus event data (white circles in FIG. 8) is generated if the luminance change toward a brighter direction exceeds a set positive threshold, and minus event data (black circles in FIG. 8) is generated if the luminance change toward a darker direction exceeds a set negative threshold. In other words, the event data image EI is independently and asynchronously acquired from the event camera 80 on a per-pixel basis as movement information that indicates movement only when a part (contour, line segment, dot, or other features) that has a luminance difference compared to its surroundings moves. When the luminance gain of the sclera WE is set to a value (luminance value) around 255 as the threshold for the plus event data, the plus event data tends to be output along the line segments of the upper and lower eyelid lines UL, LL. When the luminance gain of the pupil circle EP is set to a value (luminance value) around 0 as the threshold for the minus event data, the minus event data tends to be output along the contour of the pupil circle EP.

Accordingly, the amount of data in the event data image EI is significantly smaller than that of the anterior ocular segment image FI, in which the luminance data of all pixels is output. The frame rate of the event camera 80 exceeds 1,000 frames per second (FPS), whereas that of an ordinary anterior ocular segment camera 22 is around 60 FPS. FPS (frames per second) is a unit that represents the number of images in a video per second.

Thus, the output information from the event camera 80 includes event data information for the plus and minus event data per pixel, positional coordinate data on the two-dimensional XY coordinate plane, and time information indicating when the data was acquired. Accordingly, it becomes possible to perform the extraction process, the face movement determination process, and the eye movement determination process for moved parts in real time through high-speed processing during alignment or while capturing the subject eye image, using the output information (event data information, positional coordinate data, and time information) from the event camera 80.

In the face movement monitoring process, the face movement is determined by analyzing the movement of the extracted inner and outer canthus points IC, OC along the time axis. This is because the inner and outer canthus points IC, OC move together with the examinee's face, and the relative movement of the inner and outer canthus points IC, OC, which are positioned apart from each other, enables tracking of how the face has moved. In the eye movement monitoring process, the eye movement is determined by analyzing the movement of the extracted pupil circle EP and bright spot BS along the time axis. This is because monitoring changes in the shape of the pupil circle EP and bright spot BS allows tracking the closing or opening movement of the upper eyelid, and monitoring their positions allows tracking the movement of the pupil.

The face movement monitoring process will be described with reference to FIGS. 6 and 9 to 12. The face movement monitoring process proceeds sequentially through Step S101, Step S102, Step S103, Step S104, Step S105, Step S106, Step S107, Step S108, and Step S109 in the order shown in the flowchart of FIG. 6.

In Step S101, the inner and outer canthus points IC, OC are extracted using the output information from the event camera 80. In the first step of the extraction, the upper and lower eyelid lines UL, LL, where luminance changes occur along the line segments of the upper and lower eyelids, are extracted based on the luminance difference between each eyelid and the sclera WE of the subject eye E when the face of the examinee has moved. In the second step of the extraction, the two intersection points of the upper and lower eyelid lines UL, LL are extracted as the inner and outer canthus points IC, OC, respectively. The movement of the face is determined in Step S102 and subsequent steps by monitoring the movement of the extracted inner and outer canthus points IC, OC.

The determination of whether the chin has moved away from the chin rest will be described. In Step S102, it is assumed that the inner and outer canthus points IC', OC' move upward to the positions of the inner and outer canthus points IC, OC along the arrows on the XY coordinate plane as shown in the explanatory view of FIG. 9A. At this time, the first determination portion 642 determines that the examinee's chin J has moved away from the chin rest 30 and that the face has been displaced upward by acquiring the monitoring result indicating that the inner and outer canthus points IC, OC have moved in the same upward direction by the same amount.

In Step S102, it is assumed that the inner and outer canthus points IC', OC' move laterally to the positions of the inner and outer canthus points IC, OC along the arrows on the XY coordinate plane as shown in the explanatory view of FIG. 9B. At this time, the first determination portion 642 determines that the examinee's face have moved away from the chin rest 30 and the forehead rest 33 and that the face has been displaced leftward by acquiring the monitoring result indicating that the inner and outer canthus points IC, OC have moved in the same lateral direction by the same amount.

Step S103 is for outputting, as feedback information, the result of the determination in Step S102 that the examinee's chin J has moved away from the chin rest 30, to notify the examiner of this result. It is also possible to determine that the face has turned to the right, diagonally upward to the right, or diagonally upward to the left when the inner and outer canthus points IC, OC move in the same direction by the same amount. As is evident from FIGS. 9A and 9B, when it is determined that the examinee's chin J has moved away, the pupil circle EP and the bright spot BS also move in the same direction by the same amount as the inner and outer canthus points IC, OC. Accordingly, the determination may also be made by monitoring the pupil circle EP and the bright spot BS in addition to the inner and outer canthus points IC, OC. Each of FIGS. 9A and 9B shows an illustrative image of the anterior ocular segment, instead of the event data image EI displayed using white and black circles, as shown in FIG. 8, for a better understanding of the movement of the inner and outer canthus points IC, OC.

The determination of the examinee's face movement in the Z-axis direction will be described. In Step S104, it is assumed that the inner and outer canthus points IC', OC' move to the positions of the inner and outer canthus points IC, OC along the outward arrows on the XY coordinate plane as shown in the explanatory view of FIG. 10A. At this time, the first determination portion 642 determines that the examinee's face has moved toward the body 20 in the Z-axis direction by acquiring the monitoring result indicating that the distance L between the inner and outer canthus points IC', OC' has increased to the distance L' between the inner and outer canthus points IC, OC.

In Step S104, it is assumed that the inner and outer canthus points IC', OC' move to the positions of the inner and outer canthus points IC, OC along the inward arrows on the XY coordinate plane as shown in the explanatory view of FIG. 10B. At this time, the first determination portion 642 determines that the examinee's face has moved away from the body 20 in the Z-axis direction by acquiring the monitoring result indicating that the distance L between the inner and outer canthus points IC', OC' has decreased to the distance L"" between the inner and outer canthus points IC, OC.

Step S105 is for outputting, as feedback information, the result of the determination in Step S104 that the examinee's face has moved in the Z-axis direction, to notify the examiner of this result. Each of FIGS. 10A and 10B shows an illustrative image of the anterior ocular segment, instead of the event data image EI displayed using white and black circles, as shown in FIG. 8, for a better understanding of the movement of the inner and outer canthus points IC, OC.

The determination of the examinee's face inclination will be described. In Step S106, it is assumed that the inner and outer canthus points IC', OC' move to the positions of the inner and outer canthus points IC, OC along the right-turn arrows on the XY coordinate plane as shown in the explanatory view of FIG. 11A. At this time, the first determination portion 642 determines that the examinee's face has tilted leftward by acquiring the monitoring result indicating that the inner and outer canthus points IC, OC have rotated about the center portion CP of the subject eye E.

In Step S106, it is assumed that the inner and outer canthus points IC', OC' move to the positions of the inner and outer canthus points IC, OC along the left-turn arrows on the XY coordinate plane as shown in the explanatory view of FIG. 11B. At this time, the first determination portion 642 determines that the examinee's face has tilted rightward by acquiring the monitoring result indicating that the inner and outer canthus points IC, OC have rotated about the center portion CP of the subject eye E.

Step S107 is for outputting, as feedback information, the result of the determination in Step S106 that the examinee's face has tilted, to notify the examiner of this result. Each of FIGS. 11A and 11B shows an illustrative image of the anterior ocular segment, instead of the event data image EI displayed using white and black circles, as shown in FIG. 8, for a better understanding of the movement of the inner and outer canthus points IC, OC.

The determination of the examinee's face direction will be described. In Step S108, it is assumed that the right and left event cameras 80a, 80b in a stereo arrangement capture the inner and outer canthus points IC, OC as shown in the explanatory view of FIG. 12. At this time, a triangle can be drawn by connecting the inner canthus point IC, the position C1 of the right event camera 80a, and the position C2 of the left event camera 80b. Another triangle can also be drawn by connecting the outer canthus point OC, the position C1 of the right event camera 80a, and the position C2 of the left event camera 80b. Accordingly, the coordinate values z1, z2 of the inner and outer canthus points IC, OC can be calculated using the two-dimensional coordinate values (x1, y1) of the inner canthus point IC, the values (x2, y2) of the outer canthus point OC, known values, and trigonometric functions. The known values include the positions C1, C2 of the right and left event cameras 80a, 80b, the width W between the positions C1, C2, and the estimated angles α of the right and left event cameras 80a, 80b. This calculation process provides the three-dimensional coordinate position (x1, y1, z1) of the inner canthus point IC and the three-dimensional coordinate position (x2, y2, z2) of the outer canthus point OC. With the acquisition of the three-dimensional coordinate positions (x1, y1, z1) and (x2, y2, z2), the first determination portion 642 determines that the direction orthogonal to the line connecting the inner and outer canthus points IC, OC in a three-dimensional coordinate space is the direction of the face.

Step S109 is for outputting, as feedback information, the result of the determination of the direction of the examinee's face in Step S108, to notify the examiner of this result. FIG. 12 shows an illustrative image of the anterior ocular segment for a better understanding of the positions of the inner and outer canthus points IC, OC and illustrates the positional relationship between the right and left event cameras 80a, 80b.

The eye movement monitoring process will be described with reference to FIGS. 6, 13, and 14. The eye movement monitoring process proceeds sequentially through Step S111, Step S112, Step S113, Step S114, and Step S115 in the order shown in the flowchart of FIG. 6.

In Step S111, the pupil circle EP and the bright spot BS of the subject eye E are extracted using the output information from the event camera 80. In the extraction process of the pupil circle EP, the second extraction portion 651 extracts, using the output information from the event camera 80, a contour area where the luminance change occurs due to the luminance difference between the pupil and the cornea (black area) IR of the subject eye E, when the position of the pupil with the minimum luminance changes. In the extraction process of the bright spot BS, the second extraction portion 651 extracts, using the output information from the event camera 80, a spot area where the luminance change occurs due to its positional change, which is in accordance with the luminance change caused by the positional change of the pupil, when the visual line direction of the subject eye E changes. The movement of the eye is determined in the steps from Step S112 by monitoring the shape and movement of the extracted pupil circle EP and bright spot BS.

The determination of blinking and ptosis will be described. In Step S112, it is assumed that the pupil circle EP and the bright spot BS momentarily disappear when monitoring the shape of each extracted pupil circle EP and bright spot BS as shown in the explanatory view of FIG. 13A. At this time, the second determination portion 652 determines that the subject eye E has blinked by acquiring the monitoring result indicating that the pupil circle EP and the bright spot BS have disappeared due to the closing of the upper eyelid of the subject eye E.

In Step S112, it is assumed that the pupil circle EP and the bright spot BS remain at least partially missing when monitoring the shape of each extracted pupil circle EP and bright spot BS as shown in the explanatory view of FIG. 13B. At this time, the second determination portion 652 determines that the subject eye E has ptosis by acquiring the monitoring result indicating that the drooping of the upper eyelid of the subject eye E has caused a partial disappearance of the pupil circle EP and the bright spot BS.

Step S113 is for outputting, as feedback information, the result of the determination in Step S112 that the subject eye has blinked or has ptosis, to notify the examiner of this result. Each of FIGS. 13A and 13B shows an illustrative image of the anterior ocular segment, instead of the event data image EI displayed using white and black circles, as shown in FIG. 8, for a better understanding of the change in the shape of the pupil circle EP and the bright spot BS.

The determination of the subject eye's visual line will be described. In Step S114, it is assumed that the extracted pupil circle EP and bright spot BS move to the right in the drawing when monitoring their positional change as shown in the explanatory view of FIG. 14A. At this time, the second determination portion 652 determines that the direction of the visual line has changed from the frontward direction to the leftward direction by acquiring the monitoring result indicating their positional change.

In Step S114, it is assumed that the extracted pupil circle EP and bright spot BS move to the left in the drawing when monitoring their positional change as shown in the explanatory view of FIG. 14B. At this time, the second determination portion 652 determines that the direction of the visual line has changed from the frontward direction to the rightward direction by acquiring the monitoring result indicating their positional change.

Step S115 is for outputting, as feedback information, the result of the determination of the subject eye's visual line changes in Step S114, to notify the examiner of this result. Each of FIGS. 14A and 14B shows an illustrative image of the anterior ocular segment, instead of the event data image EI displayed using white and black circles, as shown in FIG. 8, for a better understanding of the positional change of the pupil circle EP and the bright spot BS.

The advantageous effects of the ophthalmologic apparatus A will be described.
(1) An ophthalmologic apparatus A includes an optical system 50 that is configured to acquire ocular information of a subject eye E of an examinee; a face support 30, 33 that is configured to support a face of the examinee; and a controller 60 that is configured to control at least the optical system 50 and the face support 30, 33. The ophthalmologic apparatus A further includes an event camera 80 that is installed at a position to capture an anterior ocular segment of the subject eye E, the event camera 80 being configured to output only event data, in which a luminance change has exceeded a set threshold from pixel data of an image frame of the anterior ocular segment of the subject eye E, and the event data being output together with information on coordinates and time as output information. The controller 60 includes a face movement monitoring processing portion 64 that is configured to extract inner canthus point IC and outer canthus point OC of the subject eye E by using the output information from the event camera 80 and to monitor a movement of the face of the examinee based on the extracted inner and outer canthus points IC, OC. This invention can provide ophthalmologic apparatus A that is capable of quickly checking whether the examinee's face has moved during the alignment and the ocular information acquisition through real-time processing.
(2) The face movement monitoring processing portion 64 includes a first extraction portion 641 that is configured to extract upper and lower eyelid lines UL, LL, along which a luminance change occurs on line segments of upper and lower eyelids of the subject eye E, respectively, due to a luminance difference between each of the upper and lower eyelids and sclera WE of the subject eye E, which corresponds to a white-eye region, when the face of the examinee has moved, by using the output information from the event camera 80, and to extract the inner and outer canthus points IC, OC at two intersection points of the upper and lower eyelid lines UL, LL; and a first determination portion 642 that is configured to determine the movement of the face of the examinee, based on an extraction result of the inner and outer canthus points IC, OC by the first extraction portion 641. This invention can quickly determine the movement of the examinee's face by simply monitoring the movement trajectory of the inner and outer canthus points IC, OC of the subject eye E extracted using the output information from the event camera 80.
(3) The first determination portion 642 is configured to monitor a movement of the inner and outer canthus points IC, OC extracted by the first extraction portion 641 and to determine that the face of the examinee has moved away from the face support (chin rest 30 and forehead rest 33), when the inner and outer canthus points IC, OC have moved in a same direction on a two-dimensional coordinate plane defined by an X-axis (left-right axis) and a Y-axis (vertical axis). This invention can determine that the examinee's face has moved away from the face support (chin rest 30 and forehead rest 33) when the inner and outer canthus points (IC, OC) have moved in the same direction. For example, this invention can determine that the examinee's chin (J) has moved away from the chin rest 30.
(4) The first determination portion 642 is configured to monitor a change in a distance L between the inner and outer canthus points IC, OC extracted by the first extraction portion 641, and to determine that the face of the examinee has moved closer in a front-back Z axis direction when an increase in the distance L is monitored and that the face of the examinee has moved away in the front-back Z axis direction when a decrease in the distance L is monitored. This invention can determine that the examinee's face has moved in the Z-axis direction by monitoring the change in the distance L between the inner and outer canthus points IC, OC.
(5) The first determination portion 642 is configured to monitor a movement of the inner and outer canthus points IC, OC extracted by the first extraction portion 641 with respect to a center portion CP of the subject eye E and to determine that the face of the examinee has tilted in a rotational direction when a rotational movement of the inner and outer canthus points IC, OC about the center portion CP is monitored. This invention can determine that the examinee's face has tilted by monitoring the rotational movement of the inner and outer canthus points IC, OC about the center portion CP of the subject eye E.
(6) The event camera 80 includes a right event camera 80a and a left event camera 80b in a stereo arrangement to capture the anterior ocular segment of the subject eye E from two different directions for one of the subject eyes E. The first determination portion 642 is configured to calculate three-dimensional position coordinates of each of the inner and outer canthus points IC, OC extracted by the first extraction portion 641 and to determine that a direction orthogonal to a line connecting the inner and outer canthus points IC, OC in a three-dimensional coordinate space is a direction of the face of the examinee. This invention can determine the direction of the examinee's face by acquiring the positions of the inner and outer canthus points IC, OC in a three-dimensional coordinate space using the right event camera 80a and the left event camera 80b arranged in a stereo configuration.
(7) The controller 60 further includes an eye movement monitoring processing portion 65 that is configured to extract a pupil circle EP representing a shape of a pupil of the subject eye E, and a bright spot BS that is projected onto a cornea of the subject eye E by the optical system 50, by using the output information from the event camera 80, and to monitor a movement of the subject eye E based on the pupil circle EP and the bright spot BS. This invention can quickly determine whether the examinee's eye has moved during the alignment and the ocular information acquisition through real-time processing.
(8) The eye movement monitoring processing portion 65 includes a second extraction portion 651 that is configured to extract, as the pupil circle EP, a contour area where the luminance change occurs due to a luminance difference between the pupil and the cornea IR of the subject eye E, which corresponds to an iris region, when a position of the pupil has changed, by using the output information from the event camera 80, and to extract, as the bright spot BS, a spot area where the luminance change occurs when a direction of a visual line of the subject eye E changes; and a second determination portion 652 that is configured to determine the movement of the subject eye E based on an extraction result of the pupil circle EP and the bright spot BS by the second extraction portion 651. This invention can quickly determine the movement of the subject eye E by simply monitoring changes in the shape and position of the pupil circle EP and the bright spot BS extracted by using the output information from the event camera 80.
(9) The second determination portion 652 is configured to monitor a shape of each of the pupil circle EP and the bright spot BS extracted by the second extraction portion 651, and to determine that the subject eye E has blinked when the pupil circle EP and the bright spot BS have disappeared and that the subject eye E has ptosis when the pupil circle EP and the bright spot BS are at least partially missing. This invention can determine that the subject eye E has blinked or has ptosis by monitoring the change in the shape of the pupil circle EP and the bright spot BS caused by their disappearance and missing.
(10) The second determination portion 652 is configured to monitor a positional change of the pupil circle EP and the bright spot BS extracted by the second extraction portion 651, and to determine the visual line of the subject eye E based on the positional change of the pupil circle EP and the bright spot BS. This invention can determine the visual line of the subject eye E by monitoring the positional change in the pupil circle EP and the bright spot BS.

The ophthalmologic apparatus A has been described with reference to the first embodiment shown in the drawings. However, the specific configuration of the ophthalmologic apparatus is not limited to that in the first embodiment. Design changes, additions, and modifications are permitted as long as they do not deviate from the gist of the inventions set forth in the attached claims.

The face support according to the first embodiment includes the chin rest 30 and the forehead rest 33. However, the face support is not limited to that of the first embodiment, as long as it fixedly supports the examinee's face by receiving force from the face through its contact with a part of the face. In one embodiment, the face support may include only a chin rest. In another embodiment, the face support may include only a forehead rest. In yet another embodiment, the face support may include only a cheek support. Furthermore, the face support may include two or three of the chin rest, the forehead rest, and the cheek support.

According to the first embodiment, the event camera 80 includes the right and left event cameras 80a, 80b in a stereo arrangement to capture the anterior ocular segment of one of the subject eyes from two different directions. However, the event camera is not limited to that of the first embodiment. For example, the event camera may be a monocular camera that captures the anterior ocular segment from a single direction to one of the subject eyes.

According to the first embodiment, the controller 60 includes the face movement monitoring processing portion 64, which monitors the movement of the examinee's face based on the inner and outer canthus points IC, OC, and the eye movement monitoring processing portion 65, which monitors the subject eye E based on the pupil circle EP and the bright spot BS. However, the controller 60 is not limited to one including both the face movement monitoring processing portion and the eye movement monitoring processing portion as in the first embodiment. For example, the controller may include the face movement monitoring processing portion, which monitors the movement of the examinee's face based on the inner and outer canthus points.

The ophthalmologic apparatus of the present invention is applied to the ophthalmologic apparatus A in the first embodiment, which observes, captures, and records the anterior ocular segment images, the ocular fundus images, and the ocular fundus tomographic images of the subject eye, and provides them as electronic images for diagnosis. However, the application of the ophthalmologic apparatus of the present invention is not limited to the ophthalmologic apparatus A that includes the capturing optical system to capture various images of the subject eye. The ophthalmologic apparatus of the present invention may be applied to an ophthalmologic apparatus that includes a subjective measurement optical system to subjectively measure various eye characteristics of the subject eye or an ophthalmologic apparatus that includes an objective measurement optical system to objectively measure various eye characteristics of the subject eye. In other words, the ophthalmologic apparatus of the present invention may be applied to various ophthalmologic apparatuses, as long as the ophthalmologic apparatus includes an optical system that acquires ocular information of a subject eye of an examinee, a face support that supports the examinee's face, and a controller that controls each of the optical system and the face support.

The present invention further discloses the following.
(1) An ophthalmologic apparatus including:
   an optical system that is configured to acquire ocular information of a subject eye of an examinee;
   a face support that is configured to support a face of the examinee; and
   a controller that is configured to control at least the optical system and the face support,
   wherein the ophthalmologic apparatus further includes an event camera that is installed at a position to capture an anterior ocular segment of the subject eye, the event camera being configured to output only event data, in which a luminance change has exceeded a set threshold from pixel data of an image frame of the anterior ocular segment of the subject eye, and the event data being output together with information on coordinates and time as output information,
   wherein the controller includes a face movement monitoring processing portion that is configured to extract inner canthus point and outer canthus point of the subject eye by using the output information from the event camera and to monitor a movement of the face of the examinee based on the extracted inner and outer canthus points.
(2) The ophthalmologic apparatus according to the above (1), wherein the face movement monitoring processing portion comprises:
   a first extraction portion that is configured to extract upper and lower eyelid lines, along which a luminance change occurs on line segments of upper and lower eyelids of the subject eye, respectively, due to a luminance difference between each of the upper and lower eyelids and sclera of the subject eye, which corresponds to a white-eye region, when the face of the examinee has moved, by using the output information from the event camera, and to extract the inner and outer canthus points at two intersection points of the upper and lower eyelid lines ; and
   a first determination portion that is configured to determine the movement of the face of the examinee, based on an extraction result of the inner and outer canthus points by the first extraction portion.
(3) The ophthalmologic apparatus according to the above (2), wherein the first determination portion is configured to monitor a movement of the inner and outer canthus points extracted by the first extraction portion and to determine that the face of the examinee has moved away from the face support, when the inner and outer canthus points have moved in a same direction on a two-dimensional coordinate plane defined by a left-right axis and a vertical axis.
(4) The ophthalmologic apparatus according to the above (2) or (3), wherein the first determination portion is configured to monitor a change in a distance between the inner and outer canthus points extracted by the first extraction portion, and to determine that the face of the examinee has moved closer in a front-back axis direction when an increase in the distance is monitored and that the face of the examinee has moved away in the front-back axis direction when a decrease in the distance is monitored.
(5) The ophthalmologic apparatus according to any one of the above (2) to (4), wherein the first determination portion is configured to monitor a movement of the inner and outer canthus points extracted by the first extraction portion with respect to a center portion of the subject eye and to determine that the face of the examinee has tilted in a rotational direction when a rotational movement of the inner and outer canthus points about the center portion is monitored.
(6) The ophthalmologic apparatus according to any one of the above (2) to (5), wherein the event camera comprises a right event camera and a left event camera in a stereo arrangement to capture the anterior ocular segment of the subject eye from two different directions for one of the subject eyes,
   wherein the first determination portion is configured to calculate three-dimensional position coordinates of each of the inner and outer canthus points extracted by the first extraction portion and to determine that a direction orthogonal to a line connecting the inner and outer canthus points in a three-dimensional coordinate space is a direction of the face of the examinee.
(7) The ophthalmologic apparatus according to any one of the above (1) to (6), wherein the controller further comprises an eye movement monitoring processing portion that is configured to extract a pupil circle representing a shape of a pupil of the subject eye, and a bright spot that is projected onto a cornea of the subject eye by the optical system, by using the output information from the event camera, and to monitor a movement of the subject eye based on the pupil circle and the bright spot.
(8) The ophthalmologic apparatus according to the above (7), wherein the eye movement monitoring processing portion comprises:
   a second extraction portion that is configured to extract, as the pupil circle, a contour area where the luminance change occurs due to a luminance difference between the pupil and the cornea of the subject eye, which corresponds to an iris region, when a position of the pupil has changed, by using the output information from the event camera, and to extract, as the bright spot, a spot area where the luminance change occurs when a direction of a visual line of the subject eye changes; and
   a second determination portion that is configured to determine the movement of the subject eye based on an extraction result of the pupil circle and the bright spot by the second extraction portion.
(9) The ophthalmologic apparatus according to the above (8), wherein the second determination portion is configured to monitor a shape of each of the pupil circle and the bright spot extracted by the second extraction portion, and to determine that the subject eye has blinked when the pupil circle and the bright spot have disappeared and that the subject eye has ptosis when the pupil circle and the bright spot are at least partially missing.
(10) The ophthalmologic apparatus according to the above (8) or (9), wherein the second determination portion is configured to monitor a positional change of the pupil circle and the bright spot extracted by the second extraction portion, and to determine the visual line of the subject eye based on the positional change of the pupil circle and the bright spot.

### List of Reference Signs

- A: ophthalmologic apparatus
- 10: pedestal
- 20: body
- 30: chin rest (face support)
- 33: forehead rest (face support)
- 50: optical system
- 60: controller
- 64: face movement monitoring processing portion
- 641: first extraction portion
- 642: first determination portion
- 65: eye movement monitoring processing portion
- 651: second extraction portion
- 652: second determination portion
- 80: event camera
- 80a: right event camera
- 80b: left event camera
- E: subject eye
- J: chin
- IC: inner canthus point
- OC: outer canthus point
- EP: pupil circle
- BS: bright spot

## Claims

1. An ophthalmologic apparatus (A) comprising:
an optical system (50) that is configured to acquire ocular information of a subject eye (E) of an examinee;
a face support (30, 33) that is configured to support a face of the examinee; and
a controller (60) that is configured to control at least the optical system (50) and the face support (30, 33);
wherein the ophthalmologic apparatus (A) further comprises an event camera (80) that is installed at a position to capture an anterior ocular segment of the subject eye (E), the event camera (80) being configured to output only event data, in which a luminance change has exceeded a set threshold from pixel data of an image frame of the anterior ocular segment of the subject eye (E), and the event data being output together with information on coordinates and time as output information, and
wherein the controller (60) comprises a face movement monitoring processing portion (64) that is configured to extract inner canthus point (IC) and outer canthus point (OC) of the subject eye (E) by using the output information from the event camera (80) and to monitor a movement of the face of the examinee based on the extracted inner and outer canthus points (IC, OC).

2. The ophthalmologic apparatus (A) according to claim 1, wherein the face movement monitoring processing portion (64) comprises:
a first extraction portion (641) that is configured to extract upper and lower eyelid lines (UL, LL), along which a luminance change occurs on line segments of upper and lower eyelids of the subject eye (E), respectively, due to a luminance difference between each of the upper and lower eyelids and sclera (WE) of the subject eye (E), which corresponds to a white-eye region, when the face of the examinee has moved, by using the output information from the event camera (80), and to extract the inner and outer canthus points (IC, OC) at two intersection points of the upper and lower eyelid lines (UL, LL); and
a first determination portion (642) that is configured to determine the movement of the face of the examinee, based on an extraction result of the inner and outer canthus points (IC, OC) by the first extraction portion (641).

3. The ophthalmologic apparatus (A) according to claim 2, wherein the first determination portion (642) is configured to monitor a movement of the inner and outer canthus points (IC, OC) extracted by the first extraction portion (641) and to determine that the face of the examinee has moved away from the face support (30, 33), when the inner and outer canthus points (IC, OC) have moved in a same direction on a two-dimensional coordinate plane defined by a left-right axis and a vertical axis.

4. The ophthalmologic apparatus (A) according to claim 2 or 3, wherein the first determination portion (642) is configured to monitor a change in a distance (L) between the inner and outer canthus points (IC, OC) extracted by the first extraction portion (641), and to determine that the face of the examinee has moved closer in a front-back (Z) axis direction when an increase in the distance (L) is monitored and that the face of the examinee has moved away in the front-back (Z) axis direction when a decrease in the distance (L) is monitored.

5. The ophthalmologic apparatus (A) according to any one of claims 2 to 4, wherein the first determination portion (642) is configured to monitor a movement of the inner and outer canthus points (IC, OC) extracted by the first extraction portion (641) with respect to a center portion (CP) of the subject eye (E) and to determine that the face of the examinee has tilted in a rotational direction when a rotational movement of the inner and outer canthus points (IC, OC) about the center portion (CP) is monitored.

6. The ophthalmologic apparatus (A) according to any one of claims 2 to 5, wherein the event camera (80) comprises a right event camera (80a) and a left event camera (80b) in a stereo arrangement to capture the anterior ocular segment of the subject eye (E) from two different directions for one of the subject eyes (E),
wherein the first determination portion (642) is configured to calculate three-dimensional position coordinates of each of the inner and outer canthus points (IC, OC) extracted by the first extraction portion (641) and to determine that a direction orthogonal to a line connecting the inner and outer canthus points (IC, OC) in a three-dimensional coordinate space is a direction of the face of the examinee.

7. The ophthalmologic apparatus (A) according to any one of claims 1 to 6, wherein the controller (60) further comprises an eye movement monitoring processing portion (65) that is configured to extract a pupil circle (EP) representing a shape of a pupil of the subject eye (E), and a bright spot (BS) that is projected onto a cornea of the subject eye (E) by the optical system (50), by using the output information from the event camera (80), and to monitor a movement of the subject eye (E) based on the pupil circle (EP) and the bright spot (BS).

8. The ophthalmologic apparatus (A) according to claim 7, wherein the eye movement monitoring processing portion (65) comprises:
a second extraction portion (651) that is configured to extract, as the pupil circle (EP), a contour area where the luminance change occurs due to a luminance difference between the pupil and the cornea (IR) of the subject eye (E), which corresponds to an iris region, when a position of the pupil has changed, by using the output information from the event camera (80), and to extract, as the bright spot (BS), a spot area where the luminance change occurs when a direction of a visual line of the subject eye (E) changes; and
a second determination portion (652) that is configured to determine the movement of the subject eye (E) based on an extraction result of the pupil circle (EP) and the bright spot (BS) by the second extraction portion (651).

9. The ophthalmologic apparatus (A) according to claim 8, wherein the second determination portion (652) is configured to monitor a shape of each of the pupil circle (EP) and the bright spot (BS) extracted by the second extraction portion (651), and to determine that the subject eye (E) has blinked when the pupil circle (EP) and the bright spot (BS) have disappeared and that the subject eye (E) has ptosis when the pupil circle (EP) and the bright spot (BS) are at least partially missing.

10. The ophthalmologic apparatus (A) according to claim 8 or 9, wherein the second determination portion (652) is configured to monitor a positional change of the pupil circle (EP) and the bright spot (BS) extracted by the second extraction portion (651), and to determine the visual line of the subject eye (E) based on the positional change of the pupil circle (EP) and the bright spot (BS).
